Europäisches Patentamt

European Patent Office

Office européen des brevets

(19)

(11) Publication number: **0 228 223**
**A2**

# EUROPEAN PATENT APPLICATION

(12)

(21) Application number: **86309753.1**

(22) Date of filing: **15.12.86**

(51) Int. Cl.⁴: **A 61 K  31/38, A 61 K  47/00**

(30) Priority: **16.12.85  US 809676**

(43) Date of publication of application: **08.07.87**
**Bulletin 87/28**

(84) Designated Contracting States: **AT BE CH DE FR GB IT LI NL SE**

(71) Applicant: **Cilag AG, 24 Industriestrasse, CH-6300 Zug (CH)**

(72) Inventor: **Boller, Fritz Henri, Sollgänterstrasse 41, CH-8189 Bülach (CH)**
Inventor: **Niederer, Roland Rudolph, Im Landgut, CH-8211 Stetten (CH)**

(74) Representative: **Jones, Alan John et al, CARPMAELS & RANSFORD 43 Bloomsbury Square, London, WC1A 2RA (GB)**

(54)  **Non-irritating suprofen solution.**

(57) A non-irritating aqueous suprofen solution containing essential oils and being suitable for drop application, is described.

**EP 0 228 223 A2**

ACTORUM AG

- 1 -

CCS-191

## NON-IRRITATING SUPROFEN SOLUTION

### Background of the invention:

This invention relates to a non-irritating aqueous suprofen solution which is suitable for drop application. Suprofen, which is an analgesic and anti-inflammatory compound has the formula p-(2-thenoyl)hydratropic acid. Suprofen is a white to slightly yellow microcrystalline powder having a melting point of 124.3°C. It is freely soluble in methanol, ethanol, chloroform, acetone, polyethylene glycol and 1.0 N sodium hydroxide. It is only very slightly soluble in water. Suprofen causes a very characteristic irritation in the throat, this behavior being related to the compound. The irritation is not caused by the taste of suprofen, such as bitterness, but rather it is assumed to be a specific interaction between the suprofen molecule and receptors in the mucous membrane of the throat. No irritation is observed in the mouth per se. The irritation appears only seconds after a preparation of suprofen is swallowed and thereafter it proceeds to the oesophagus and then disappears within minutes. The repetition of this procedure seems to reduce the sensitivity. The irritation effect occurs in relation to suprofen acid as well as to all known salts of suprofen such as suprofen-sodium. In view of this irritation effect, perorally used dosage forms are only practical if the suprofen, per se, does not come in contact with the mucous membrane of the throat. Such contact can be prevented if suprofen is incorporated in a gelatin capsule

0228223

- 2 -

or a film tablet. In addition, suprofen or suprofen salts dissolved in liquid dosage forms are acceptable if the suprofen concentration is no greater than 20 mg/ml and providing that the viscosity is increased by means of thickening agents such as polyvinylpyrrolidone (although, even in this instance, a slight irritation may occur). The only liquid perorally used dosage form which is acceptable is a syrup in which the suprofen concentration is generally about 2%. In this instance, the therapeutic single dose of suprofen is preferably between 100 and 200 mg.

Heretofore, it has not been feasible to administer suprofen in the form of drops, in view of the fact that a single dose in drop form should not consist of more than 1 ml of a preparation and thickening agents which have been used up to the present time, prevent a correct dosage of drops. This is due to the fact that the viscosity of suitable drops should range between that of alcohol and water.

Drops are the preferred dosage form for children and elderly patients. Drops provided for children should, of course, be alcohol free.

In accordance with the present invention, there is provided a suprofen containing composition which is especially suitable for drop application and which causes a negligible amount of irritation in the mucous membrane of the throat.

Nowhere in the prior art is there any disclosure of any method of inhibiting suprofen-caused irritation of the mucous membrane of the throat, in any compositions suitable for drop application (in a composition having a viscosity in the range between that of alcohol and water).

Hayes in U.S. Patent No. 4,423,030 discloses a mouthwash vehicle having dispersed therein a two-tone flavor comprising essential oil and water-insoluble oleoresin extract of dried fruit. The Hayes mouthwash is, of course, not intended for peroral administration of a drug and the problem of the inhibition of suprofen-caused irritation of the mucous membrane of the throat is obviously not addressed. The essential oil is used by Hayes purely for the purpose of enhancing the flavor.

Essential oils are referred to in the following patents:

Japanese Patent No. 56-38394; U. S. Patent Nos. 2,001,046; 2,196,322; 2,311,923; 3,920,816; 3,950,519; 3,989,823; 4,402,950 and 4,423,030.

These references disclose the use of many essential oils as fragrances and, in addition, certain essential oils are used for therapeutic purposes. However, there is no disclosure in any of said patents concerning the inhibition of suprofen-caused irritation of the mucous membrane of the throat with high concentrations of various essential oils, such as is provided in accordance with the present invention.

Summary of the Invention

In accordance with the objects and principles of the present invention, there is provided an aqueous peroral composition especially suited for drop application comprising:

(a) About 2 - 30% by weight of suprofen;

(b) about 0.3 to 0.5% by weight of a water soluble, non-toxic base;

(c) about 0.1 to 20% by weight of at least one pharmaceutically acceptable essential oil;

(d) about 2 to 70% by weight of a solubilizing agent for rendering said essential oils soluble in said aqueous composition, with the proviso that said composition comprises at least 10% by weight of water. The composition of the

CCS-191

invention preferably contains anethol, which is present in anise oil or fennel oil; although clove oil, cinammon oil or citrus oil are also suitable.

Detailed Description of the Invention:

In accordance with the invention there is provided a suprofen composition which includes essential oils, preferably containing anethol, which is present, for example, in anise oil and fennel oil. Pure anethol may also be used. In addition, anethol-free essential oils such as clove oil, cinammon oil and citrus oil also inhibit the suprofen-caused irritation of the mucous membrane of the throat.

In accordance with the present invention, the expression "essential oil" refers to volatile oils distilled from plants, distinguished from fatty oils by their volatility, non-greasiness and non-saponifying properties. "Essential oil" includes oil from various parts of plants, such as the fruit, leaves, twigs or flowers, parts other than the fruit itself being preferred. Further, the term includes the natural derivatives of such essential oils such as menthol, anethol, eucalyptol, carvone, eugenol, isoeugenol, terpenols, terpenes, terpinenes, and terpinones. Typical essential oils are peppermint oil, spearmint oil, clove oil, sassafras oil, aniseed oil, cinammon oil, eucalyptus oil, wintergreen oil, cassia oil, cardamon oil, orris oil, rose oil, geranium oil and thyme oil. Mixtures of said oils may also be used.

The composition of the present invention may contain from about 0.1 to 20% by weight of at least one pharmaceutically acceptable essential oil, but the preferred range of essential oil is between about 0.5 to 5% by weight of the composition. The amount of essential oil used is made to vary, of course, in accordance with the actual concentration of suprofen (20 mg/ml-300 mg/ml). It should also be borne in mind that the acceptable daily intake of essential oils must also be considered in preparing the composition of the invention.

In view of the fact that all essential oils are poorly soluble in water, clear aqueous solutions are obtained by adding organic solvents, such as ethanol or solubilizers such as a surfactant, e.g. polysorbate, to the composition. Numerous surfactants may be used for this purpose. These surfactants may be anionic, nonionic, ampholytic or cationic in nature. One suitable absorbent is polysorbate 80 which is polyoxyethylene (20) sorbitan mono-oleate. It consists of an oleate ester of sorbitol and its anhydrides copolymerized with approximately 20 moles of ethylene oxide for each mole of sorbitol and sorbitol anhydrides.

Another suitable surfactant is polysorbate 20 (which consists of approximately 97% of the mixture of the esters of lauric acid with sorbitol and its mono- and dianhydrides, having an acid value below 7 and a water content below 0.2% and condensed with approximately 20 moles of ethylene oxide per mole of sorbitol.

Suprofen is poorly soluble in water so that water-soluble, non-toxic bases are added to the composition to improve the solubility of suprofen in this connection. Although any water soluble non-toxic base may be used, the preferred base is sodium hydroxide and the pH of the final product should be between 5 and 9, and preferably between 6 and 8. The base is preferably present in a concentration between about 0.3 and 5% by weight.

Propylene glycol often assists in the solubilization of suprofen. In the instance where an ethanol is used to improve the solubility of the essential oils, less surfactant need be used for this purpose.

A further surfactant, suitable for use in accordance with the present invention, is poloxamer 407. The poloxamers are polyethylenepolypropylene glycols, the molecular weight ranging from 1,000 to greater than 16,000.

The pharmaceutical composition of the present invention, useful for adults as an analgesic in unit peroral dosage form, comprises about 1 ml. of the aforementioned composition, in the instance wherein said composition comprises about 20% by weight of suprofen. When the pharmaceutical composition of the present invention is administered to children as an analgesic in unit peroral dosage form, it comprises about 1 ml. of the aforementioned composition, in the event that the composition contains about 10% by weight of suprofen.

When the composition of the invention is administered in drop form, about 20 drops are equal to 1 ml. in volume. Thus, about 200 mg. of suprofen per ml. are required for adult administration and about 100 mg. of suprofen per ml. are required for administration to children.

A preferred suprofen composition for administration to adults includes sodium hydroxide, anise oil, polysorbate 80, ethanol, propylene glycol and water. A preferred suprofen composition for administration to children, includes sodium hydroxide, anise oil, polysorbate 20, poloxamer 407 and water.

The composition of the present invention may include small amounts of other agents, not strictly essential for the purposes of the invention, such as sweeteners, preservatives and dyes.

In vivo studies were carried out with the suprofen formulations of the invention as follows:

|  | Strength | Number |
|---|---|---|
| Bioavailability | 200 mg/ml | 24 adult volunteers |
|  | 100 mg/ml | 12 adult volunteers |
| Effectiveness | 200 mg/ml | 50 adult patients |
|  | 100 mg/ml | 112 children |

The formulation of the following Example 1 was used for administration to the children; and the formulation of the following Example 2 was used for administration to the adults. No irritation in the throat was reported.

Bioavailability is determined by assaying the amount of suprofen in the blood, whereas the effectiveness is a measure of how effective the formulation is against pain and inflammation. Both the bioavailability and effectiveness of the formulations of following Examples 1 and 2 were determined to be good.

The invention may be put into practice in various ways and a number of specific embodiments will be described to illustrate the invention with reference to the accompanying examples. All amounts given are mg. per ml. of the total composition. The compositions of the following examples are formulated at room temperature as follows:

Water is placed in a suitable vessel and the water-soluble base is added. Suprofen is then added and dissolved and then a mixture of all the other ingredients (such as the essential oil, the organic solvent and the surfactant) are added and well mixed.

– 8 –

## Example 1

| | mg/ml |
|---|---|
| Suprofen | 100 |
| Sodium hydroxide | 15 |
| Anise oil | 5 |
| Polysorbate 20 | 20 |
| Poloxamer 407 | 25 |
| Water | 835 |

## Example 2

| | mg/ml |
|---|---|
| suprofen | 200 |
| Sodium hydroxide | 30 |
| Anise oil | 50 |
| Polysorbate 80 | 25 |
| Ethanol | 400 |
| Water | 175 |
| Propylene glycol | 120 |

## Example 3

| | mg/ml |
|---|---|
| Suprofen | 200 |
| Sodium hydroxide | 30 |
| Fennel oil | 50 |
| Polysorbate 80 | 100 |
| Ethanol | 350 |
| Propylene glycol | 170 |
| Water | 100 |

## Claims

1. An aqueous peroral composition especially suitable for drop application, comprising :

    a)    about 2 to 30% by weight of suprofen,

    b)    about 0.3 to 5% by weight of a water soluble, non-toxic base;

    c)    about 0.1 to 20% by weight of at least one pharmaceutically acceptable essential oil; and

    d)    about 2 to 70% by weight of a solubilizing agent for rendering said essential oil soluble in said aqueous composition, with the proviso that said composition comprises at least 10% by weight of water.

2. The composition of claim 1, in which said essential oil contains anethol.

3. The composition of claim 1 or claim 2, in which said essential oil comprises anise oil or fennel oil.

4. The composition of claim 1, in which said essential oil comprises clove oil, cinammon oil or citrus oil.

5. The composition of any one of claims 1 to 4, in which the pH is between 5 and 9.

6. The composition of claim 5, in which the pH is between 6 and 8.

7. The composition of any one of claims 1 to 6, in which the said essential oil comprises about 0.5 to 5% by weight thereof.

8. The composition of any one of claims 1 to 7, wherein said solubilizing agent comprises an organic solvent.

9. The composition of any one of claims 1 to 7, wherein said solubilizing agent comprises a surfactant.

10. The composition of any of claims 1 to 7, wherein said solubilizing agent comprises propylene glycol.

11. The composition of any one of claims 1 to 7, wherein said solubilizing agent comprises a mixture of an organic solvent and a surfactant.

12. The composition of claim 8 or claim 11, wherein said organic solvent comprises ethanol.

13. The composition of claim 9 or claim 11, wherein said solubilizing agent comprises polysorbate 80.

14. A pharmaceutical composition, useful for adults as an analgesic in unit peroral dosage form, comprising about 1 ml of the composition of any one of claims 1 to 13, said composition containing about 20% by weight of suprofen.

15. The composition of claim 14, said composition including sodium hydroxide, anise oil, Polysorbate 80, ethanol and propylene glycol.

16. A pharmaceutical composition, useful for children as an analgesic in unit peroral dosage form, comprising about 1 ml of the composition of any one of claims 1 to 13, said compostion containing about 10% by weight of suprofen.

17. The composition of claim 15, said composition including sodium hydroxide, anise oil, Polysorbate 20, and Poloxamer 407.

18. A method for producing a composition according to any one of claims 1 to 17 comprising mixing together:

a) about 2 to 30% by weight of suprofen,

b) about 0.3 to 5% by weight of a water soluble, non-toxic base;

c) about 0.1 to 20% by weight of at least one pharmaceutically acceptable essential oil; and

d) about 2 to 70% by weight of a solubilizing agent for rendering said essential oil soluble in said aqueous composition.

to form a composition comprising at least 10% by weight of water.